# EUROPEAN PATENT SPECIFICATION

(11) **EP 2 154 382 B1**
(45) Date of publication and mention of the grant of the patent: **26.06.2019**
(21) Application number: 08752275.1
(22) Date of filing: 01.05.2008
(51) Int. Cl.: F16B 2/12, A61M 5/00, A61M 5/14

(54) **CLAMP DEVICE**
KLEMMVORRICHTUNG
DISPOSITIF DE SERRAGE

(30) Priority: 08.05.2007 JP 2007122984
(43) Date of publication of application: 17.02.2010
(73) Proprietor: Terumo Kabushiki Kaisha, Tokyo 151-0072 (JP)
(72) Inventor: KURIMOTO, Masuya, Fujinomiya-shi Shizuoka 418-0015 (JP); NAKANISHI, Masaru, Fujinomiya-shi Shizuoka 418-0015 (JP); HASEGAWA, Makoto, Fujinomiya-shi Shizuoka 418-0015 (JP)
(74) Representative: Mewburn Ellis LLP
(86) International application number: PCT/JP2008/058345
(87) International publication number: WO 2008/139951

(56) References cited:
- EP-A2- 1 988 298
- WO-A1-2008/090068
- JP-U- 03 119 604
- JP-U- 03 122 842
- JP-U- H03 119 604
- US-A1- 2006 233 601

## Description

### TECHNICAL FIELD

The present invention relates to a clamp apparatus and, more particularly, to a clamp apparatus for detachably fixing various types of infusion devices to infusion stands attached with poles having different diameters.

### BACKGROUND ART

Infusion devices used in medical sites include a peristaltic infusion pump which causes peristaltic motion of an infusion tube to perform infusion and a syringe pump which performs accurate infusion by using a syringe (patent reference 1). When performing infusion using such an infusion device, it is necessary to fix the infusion device to a pole fixed upright on an infusion stand by using a clamp apparatus. In general, a clamp apparatus is provided with a mount base. Fixing a peristaltic infusion pump or a syringe pump on the mount base will fix the infusion pump or the syringe pump on the infusion stand.

In some cases, a plurality of infusion devices are moved by fixing an infusion pump and a syringe pump to the pole of a common infusion stand. This allows carrying of a specific patient even during infusion.
In addition, various types of infusion devices each are often detached from a given infusion stand and fixed to another infusion stand for medical treatment for another patient. This attaches considerable importance to the operability of the clamp apparatus with respect to the infusion stand.

A conventional clamp apparatus includes a clamp base obtained by integrally forming a grip-shaped portion which grips one outer peripheral surface of a pole and an upright shaped portion provided at a position to face the grip-shaped portion, a main shaft member having a distal end provided with a grip member which grips the other outer peripheral surface of the pole, and a rotating knob fixed to the other end of the main shaft member. Pivoting the rotating knob clockwise or counterclockwise can fix each type of infusion device to the pole of each type of infusion stand and detach the device from the pole.

A turnbuckle used to assemble a shell on a building site or the like is used by being threadably engaged between two rods. More specifically, pivoting the turnbuckle at the time of assembly will shorten the distance between the respective rods and hold them in a state of tension. Pivoting the turnbuckle reversely at the time of disassembly will increase the distance between the respective rods. Therefore, with an increase in the amount of change in distance between the respective rods, the amount of pivoting operation increases. Under the circumstance, a one-touch turnbuckle capable of achieving a desired amount of change with one touch has been proposed (patent reference 2).
Patent reference 1: Japanese Patent No. 3502086
Patent reference 2: Japanese Patent Laid-Open No. 2003-314517
   JP H03119604 proposes a pole clamp equipment.

### DISCLOSURE OF INVENTION

### PROBLEMS THAT THE INVENTION IS TO SOLVE

The conventional clamp apparatus described above, however, has been configured to set the pole of the infusion stand in a gripped state or an ungripped state by rotating the rotating knob to move the main shaft member at the time of attachment/detachment to/from the pole. For this reason, when attaching an infusion device to an infusion stand whose pole has a different diameter, the user needs to hold the infusion device with one hand so as not to drop it and, at the same time, rotate the rotating knob a plurality of number of times with the other hand. When detaching the device, the user needs to perform the reverse operation.

It takes much time to attach an infusion device to the pole of an infusion stand and detach the device from the pole in this manner. In addition, this places a heavy physical load on a nurse. For these reasons, demands have arisen for an improvement in this technique especially in medical sites, which often include emergency cases.

The present invention has been made in consideration of the above situation, and has as its object to provide a clamp apparatus which implements the operation of attaching an infusion device to the pole of an infusion stand and detaching the device from the pole in a short period of time with a light physical load.

### MEANS OF SOLVING THE PROBLEMS

In order to solve the above problems, a clamp apparatus according to the present invention is provided according to claim 1.

In addition, this apparatus is characterized in that the housing portion of the main shaft member is formed as a through-space portion along a longitudinal direction of the main shaft member, a pair of first inclined surfaces are formed on a left side surface side of the through-space portion so as to be symmetric with respect to a direction extending from the outer peripheral surface of the main shaft member to the other end of the main shaft member, and a first projection is formed on a right side surface side of the through-space portion, male thread portions respectively meshed with the female thread portions, a pair of second inclined surfaces which slide on the pair of first inclined surfaces, and second projections are integrally formed on the pair of male thread pieces, a built-in spring is provided between the first projection and the second projections to bias the pair of male thread pieces in a direction toward the female thread portions, and the operation means comprises a tubular member which covers the outer peripheral surface of the main shaft member and in which a pair of opening portions for setting the pair of male thread pieces in the meshed state are formed, and a return spring which biases the operation portion in a direction toward the separate position.

This apparatus is characterized in that first abutment surfaces are formed on left side surfaces of the pair of opening portions of the tubular member, second abutment surfaces which come into contact with the first abutment surfaces from left end sides of the female thread portions to the first abutment surfaces are formed in the pair of male thread pieces, and the first abutment surfaces come into contact with the second abutment surfaces, immediately after the operation portion moves to the operation position, to maintain the meshed state, and the unmeshed state is set when the rotating knob is rotated afterward.

This apparatus is characterized in that the first abutment surfaces and the second abutment surfaces are respectively formed within planes perpendicular to an axis of the main shaft member.

This apparatus is characterized in that a first block member on which the pair of first inclined surfaces are formed and a second block member on which the first projection is formed are discrete, and the first block member and the second block member are set in the through-space portion, together with the pair of male thread pieces and the built-in spring.

This apparatus is characterized in that a mount base for an infusion device is used while being fixed on the clamp base.

This apparatus is characterized in that the operation means comprises a boot cover made of an elastic material which prevents entrance of a drug by covering the tubular screw member.

This apparatus is characterized in that the clamp base is used while being directly fixed to a rear surface of an infusion device.

### EFFECTS OF THE INVENTION

According to the clamp apparatus of the present invention, it is not necessary to repeatedly and continuously rotate the rotating knob while holding an infusion device when the infusion device is fixed to the pole of the infusion stand or detached from the pole. This greatly simplifies and facilitates the operation of fixing an infusion device to the pole of the infusion stand and the operation of detaching the device from the pole, thereby minimizing the time loss and physical load in the operation.

In addition, immediately after the operation portion is moved to the operation position, the meshed state is maintained. An unmeshed state is set only when the rotating knob is rotated. This makes it unnecessary to hold the infusion device, and makes it possible to safely and reliably detach the infusion device from the pole of the infusion stand. That is, even when the operation portion is moved to the operation position, the meshed state is maintained, and the unmeshed state is set only when the rotating knob is rotated. This makes it possible to safely maintain the fixed state.

Other features and advantages of the present invention will be apparent from the following description taken in conjunction with the accompanying drawings, in which like reference characters designate the same or similar parts throughout the figures thereof.

### BRIEF DESCRIPTION OF DRAWINGS

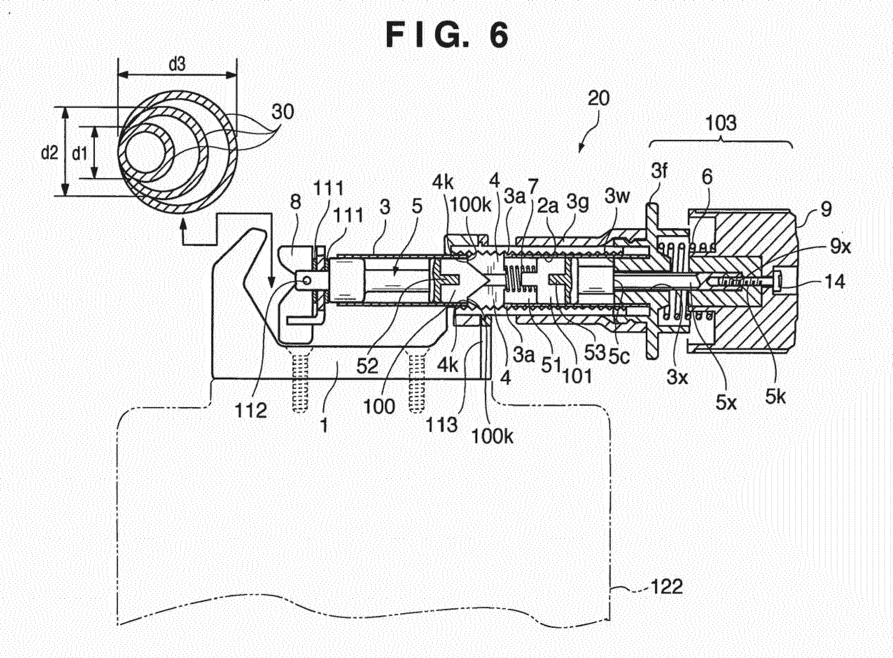

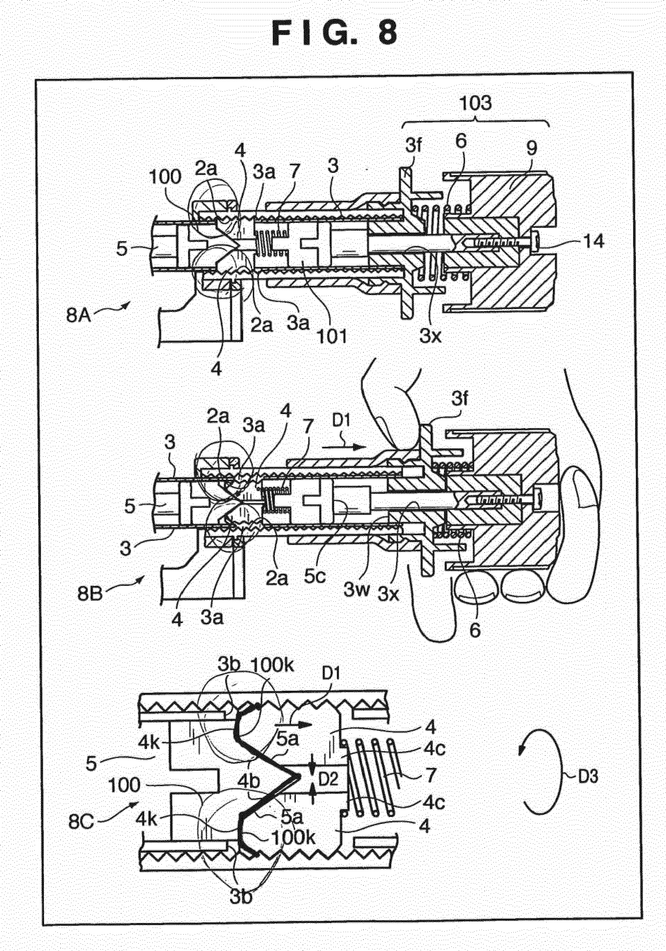

The accompanying drawings, which are incorporated in and constitute a part of the specification, illustrate embodiments of the invention and, together with the description, serve to explain the principles of the invention.
Fig. 1 is a perspective view of an outer appearance showing an operation state in which a clamp apparatus according to an embodiment of the present invention is fixed to the pole of an infusion stand for an infusion device;
2A of Fig. 2 is a sectional view taken along a line X - X of the clamp apparatus in Fig. 1, and 2B of Fig. 2 is a sectional perspective view of the main part of the apparatus;
Fig. 3 is a perspective view of the outer appearance of the clamp apparatus according to an embodiment of the present invention;
Fig. 4 is an exploded perspective view of the arrangement of the main part of the clamp apparatus according to an embodiment of the present invention;
5A of Fig. 5 is a sectional view of the main part, showing a gripped state for an explanation of operation, 5B of Fig. 5 is a sectional view of the main part, showing a midway state, and 5C of Fig. 5 is a sectional view of the main part, showing a moving state for an explanation of operation;
Fig. 6 is a sectional view of the main part, showing how a clamp apparatus 20 according to another embodiment of the present invention is directly fixed to the rear surface of a peristaltic infusion device 122;
Fig. 7 is an exploded perspective view of the clamp apparatus 20 in Fig. 6;
8A of Fig. 8 is a sectional view of the main part in Fig. 6, showing how a pair of male thread pieces 4 are meshed with female thread portions 2a, respectively, 8B of Fig. 8 is a sectional view of the main part in Fig. 6, showing how the pair of male thread pieces 4 are unmeshed from the female thread portions 2a, respectively, by slightly rotating a rotating knob 9 counterclockwise, and then moving the operation portion 3f in the direction indicated by the arrow D1, and 8C of Fig. 8 is an enlarged sectional view immediately after the operation portion 3f of the operation means 103 is moved in the direction indicated by the arrow D1; and

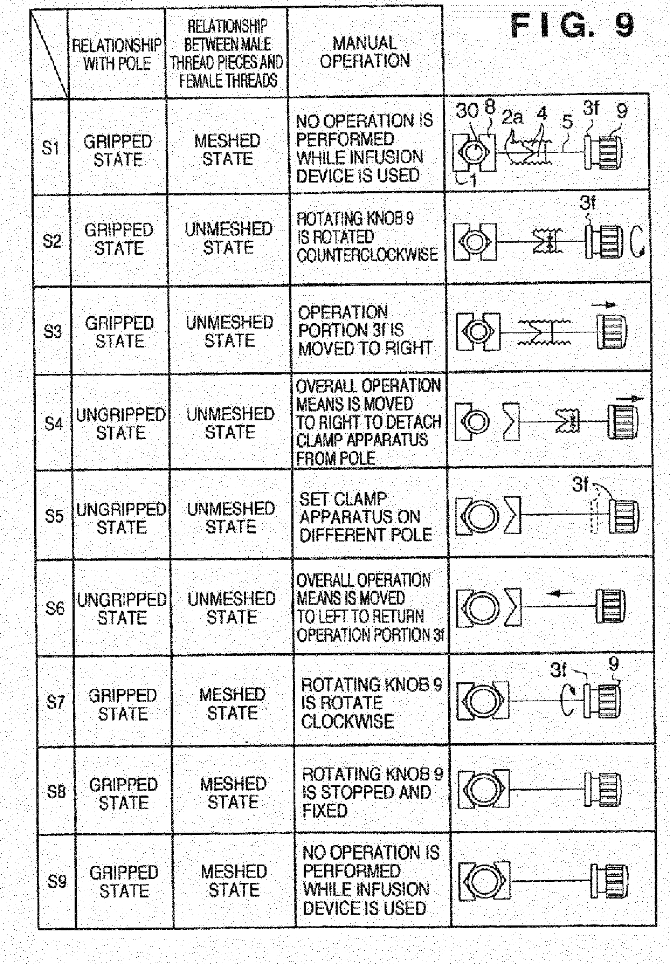

Fig. 9 is a schematic view showing the relationship between a pole 30 and a grip member 8, the relationship between the male thread pieces 4 and the female thread portions 2a, the contents of manual operation, and the states of the clamp apparatus along with the sequence of steps S1 to S9.

### BEST MODE FOR CARRYING OUT THE INVENTION

A preferred embodiment of the present invention will be described below with reference to the accompanying drawings. Note that the following description will mainly focus on the evaluation made by making alterations to a conventional clamp apparatus. Obviously, however, the present invention is not limited to this, and various arrangements defined in the scope of the following claims are available. In addition, the following description will mainly focus on an example of how a syringe pump is used as an infusion device. Obviously, however, the present invention is not limited to this, and can also be applied to various types of infusion devices including a peristaltic finger-type infusion pump configured to cause peristaltic motion of an infusion tube and a roller type infusion pump including a rotating roller as an infusion device of another type.

Furthermore, although not recommended much in consideration of safety, for example, an infusion device can be arbitrarily fixed to something other than the pole of the infusion stand in an emergency.

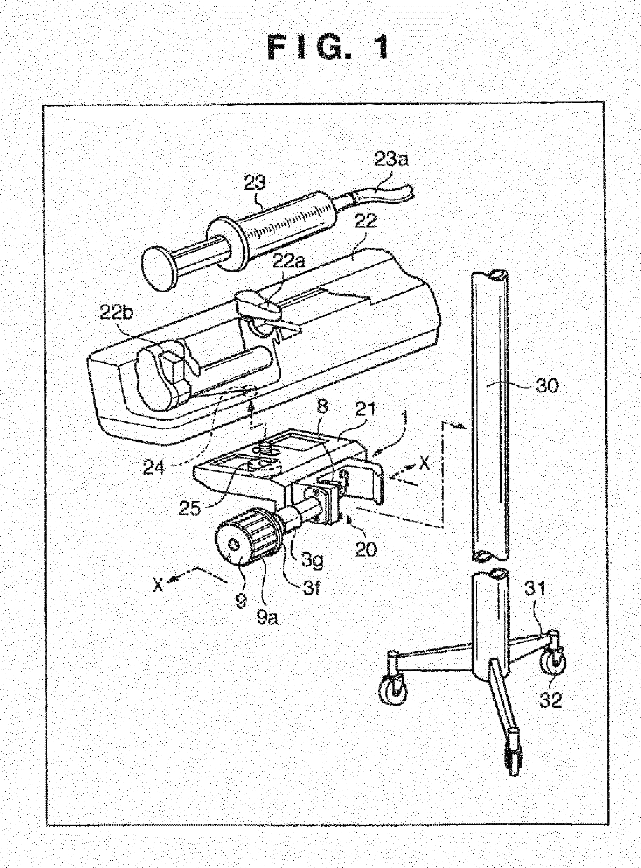

Fig. 1 is a perspective view showing the outer arrangement of a clamp apparatus 20 according to an embodiment of the present invention, the outer arrangement of a syringe pump 22 which is an infusion device using a syringe 23, and the outer arrangement of a pole 30 of an infusion stand. As shown in Fig. 1, the syringe pump 22 is provided with a member 22a to which the main body of the syringe 23 is fixed and a slider 22b which grips the plunger. When the slider 22b grips the plunger pulled out to the maximum position and a built-in mechanism slides the slider 22b, the syringe 23 accurately feeds a drum through an infusion tube 23a.

The bottom surface of the syringe pump 22 is provided with a female thread hole 24 shown in Fig. 1 by the broken line. While the syringe pump 22 is placed on a mount base 21, a set screw 25 provided on the mount base 21 is threadably engaged with the female thread hole 24 to fix the syringe pump 22 on the mount base 21. Alternatively, it is possible to fix the syringe pump 22 on the mount base 21 by engaging the mount base 21 with the clamp apparatus 20 using a screw in advance, fixing the clamp apparatus 20 to the pole 30, placing the syringe pump 22 on the mount base 21, and then threadably engaging the set screw 25 with the female thread hole 24. Note that since the mount base 21 slightly tilts forward as shown in Fig. 1, the user can, for example, easily attach/detach the syringe 23. Note that it is possible to use the clamp apparatus 20 while it is directly fixed on the rear surface of an infusion device as will be described later.

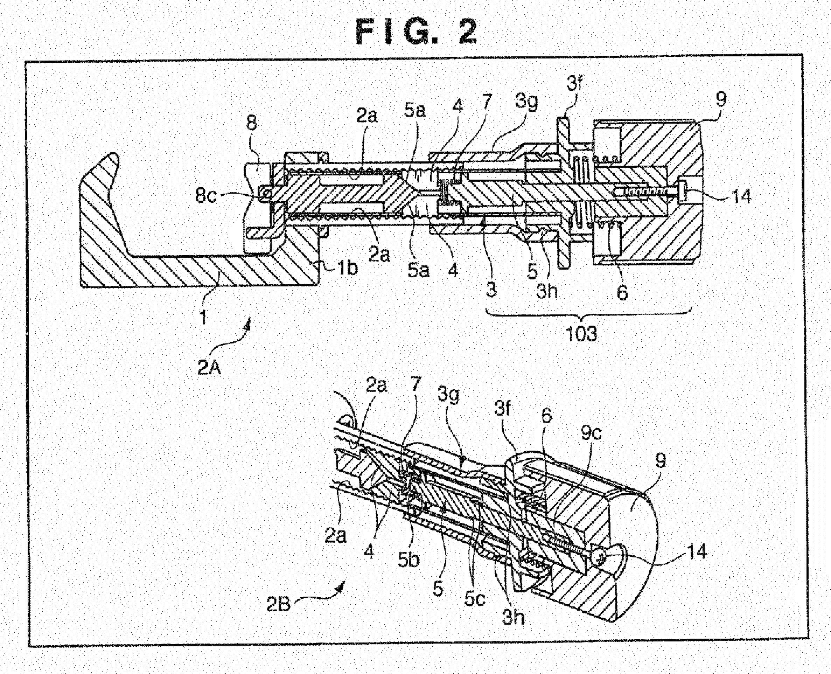

A clamp base 1 of the clamp apparatus 20 is fixed on the mount base 21 by using four screws. The clamp base 1 is provided with a main shaft member 5 (see Figs. 2, 4, 5, 6, 7, 8, and 9) as will be described later. A rotating knob 9 is fixed to one end of the main shaft member 5. A grip member 8 is rotatably provided on the other end of the main shaft member 5. In order to prevent a drug or the like from entering the operation means, the main shaft member 5 is provided with a boot cover 3g formed by a thermoplastic material having excellent chemical resistance, for example, silicone rubber as shown in Fig. 1. In addition, an operation portion 3f is provided adjacent to the rotating knob 9. The rotating knob 9 is provided with a nonslip groove portion 9a in the axial direction.

The pole 30 of the infusion stand is fixed to leg portions 31 provided with moving casters 32, and extend in a direction perpendicular to a floor surface. The pole 30 is formed from a metal pipe, and varies in diameter for each type of device.

The clamp apparatus 20 will be described below, which has the above arrangement and allows to easily fix the syringe pump 22 to the pole 30 of the infusion stand and detach the syringe pump 22 from the pole 30 of the infusion stand by only rotating the rotating knob 9 to a minimum necessary extent.

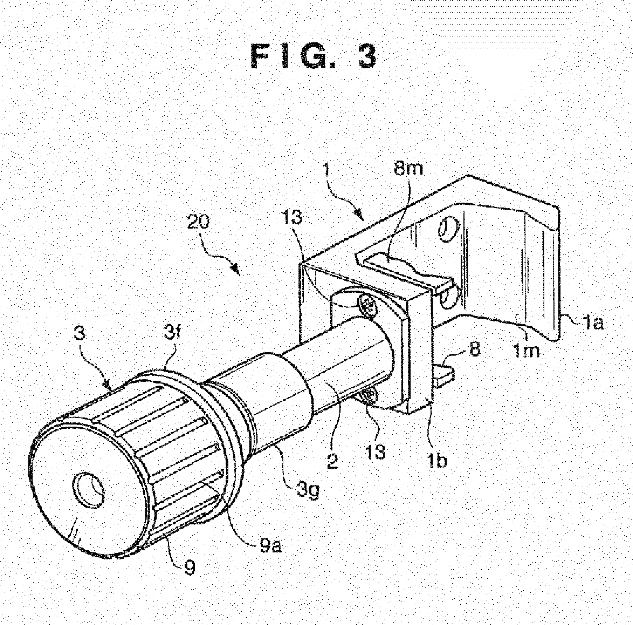

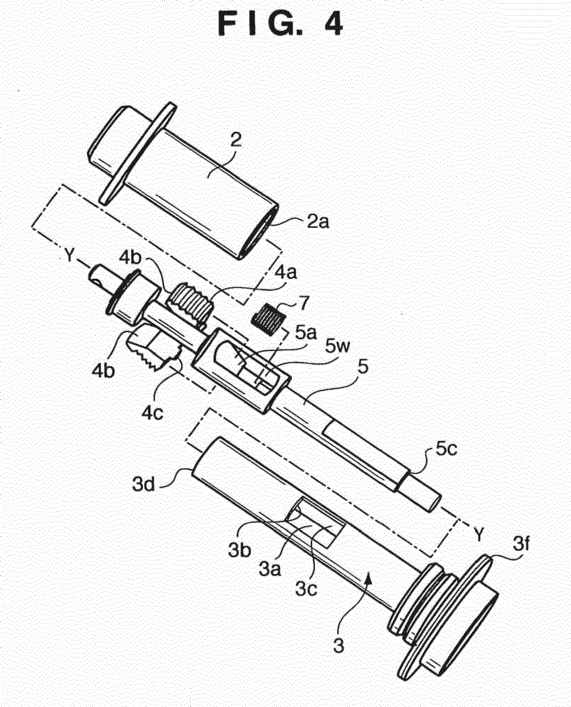

2A of Fig. 2 is a sectional view taken along a line X - X of the clamp apparatus 20 in Fig. 1. 2B of Fig. 2 is a sectional perspective view of part of the apparatus. Fig. 3 is a perspective view of the outer appearance of the clamp apparatus 20. Fig. 4 is an exploded perspective view of the arrangement of the main part of the clamp apparatus 20.

First of all, referring to 2A of Fig. 2, the clamp apparatus 20 has a total length of about 190 mm, and the clamp base 1 has a width of 75 mm and is configured to be used for the pole 30 whose diameter falls within the diameter range of 12.5 to 3534 mm.
The clamp apparatus 20 has a total weight of 200 to 350 g. Obviously, these dimensions are merely examples, and dimension setting is properly performed in accordance with the size or the like of an infusion device.

A tubular thread member 2a having female thread portions 2a formed on the inner peripheral surface is fixed to an upright shaped portion 1b of the clamp base 1. The rotating knob 9 is fixed to the main shaft member 5 with a screw 14. A return coil spring 6 is provided between the rotating knob 9 and the operation portion 3f. The rotating torque generated by rotating the rotating knob 9 is transmitted to the main shaft member 5 as a hole portion 9c of a D-cut member formed in the rotating knob 9 by insert molding is meshed with the main shaft member 5 having a D-cut.

The main shaft member 5 is made of a stainless steel rod or the like and has inclined surfaces 5a which are symmetrically formed at opposite positions. A pair of male thread pieces 4 are accommodated in the main shaft member 5. The pair of male thread pieces 4 are biased by a built-in spring coil 7. This makes it possible for the male thread pieces 4 to slide and move on the inclined surfaces. Likewise, the inner surface of the tubular thread member 2, which is a cylindrical member made of stainless steel, is provided with the female thread portions 2a almost throughout the total length in the longitudinal direction. Small-diameter portions are formed at two portions of the main shaft member 5, as shown in Fig. 2. This reduces the resistance produced when a tubular member 3 (to be described later) slidably moves.

Rectangular opening portions 3a are vertically provided midway along the tubular member 3 covering the main shaft member 5. This causes the pair of male thread pieces 4 described above to protrude toward the female thread portions 2a vertically positioned as shown in Fig. 2. In this case, Fig. 2 shows a case in which the opening portions 3a are vertically positioned. Obviously, however, as the tubular member 3 rotates,
the positions of the opening portions 3a change. The flange-shaped operation portion 3f is fixed to an operation means 103 provided with the tubular member 3. That is, as shown in Fig. 4 which is an exploded perspective view of the arrangement of the main part, the operation means 103 has an inner diameter portion 3c larger than the outer diameter of the main shaft member 5, and each abutment surface 3b is formed in each opening portion 3a. The pair of male thread pieces 4 each are made of a stainless resin, metal, or a material having similar strength, and include a male thread portion 4a which is meshed with the corresponding female thread portion 2a, an inclined surface 4b continuously formed from the corresponding male thread portion 4a as shown in Fig. 4, and a projection 4c accommodating the built-in coil spring 7. The male thread pieces 4 and the built-in coil spring 7 are built in a housing portion 5w of the main shaft member 5. With this arrangement, the main shaft member 5 is biased.

Each male thread portion 4a is formed from a single-, double-, or multi-start thread, and is preferably formed from a single-start thread which greatly moves upon slight rotation in the horizontal direction, and has larger fixing force than double- and multi-start threads. In this case, the built-in coil spring 7 has an outer diameter of 3.55 to 10 mm, a length of 10 to 14 mm, and a spring modulus of 16 to 30 kgf/mm. If the spring modulus is smaller than 10 kgf/mm, the pressing force against the male thread pieces 4 becomes small. If the spring modulus is larger than 30 kgf/mm, it is difficult to release each male thread piece 4 from the corresponding female thread portion 2a. Note that an escape prevention rib 3h is formed on the cover 3g. In addition, a projection 5b is inserted into one end side of the built-in coil spring 7, and functions to permit only the movement of the built-in coil spring 7 in the longitudinal direction. Furthermore, a stepped portion 5c forms a stepped surface for stopping movement of the operation portion 3f with respect to the main shaft member 5.

A more detailed description will be made with reference to Fig. 3. The clamp base 1 is formed by cutting an extruded aluminum member. A grip-shaped portion 1a and the upright shaped portion 1b are integrally formed as shown in Fig. 3. A first valley-shaped portion 1m for gripping one outer peripheral surface of the pole is formed on the grip-shaped portion 1a. The upright shaped portion 1b holds the grip member 8 on which a second valley-shaped portion 8m is formed symmetrically with respect to the first valley-shaped portion 1m.

In order to grip the other outer peripheral surface of the pole 30, the grip member 8 is placed at a distal end 8c of the main shaft member 5 having an outer diameter of about 4 to 20 mm so as to be pivotal within a predetermined range and movable in the longitudinal direction. The rotating knob 9 is fixed to the other end of the main shaft member 5. A plurality of nonslip groove portions 9a extending in the longitudinal direction are formed in the rotating knob 9 in the circumferential direction.

By operating the rotating knob 9, the user can easily fix various types of infusion devices including the syringe pump 22 described above to various types of infusion stands including the pole 30 and easily detach the devices from the various types of infusion stands.

The flange-shaped operation portion 3f described above is located near the rotating knob 9.
By operating the operation portion 3f in the longitudinal direction while gripping the rotating knob 9, the user can move the main shaft member 5 in the longitudinal direction. This makes it possible to fix the main shaft member 5 at an arbitrary position relative to the upright shaped portion 1b. Operating the rotating knob 9 in this state allows to grip the pole 30 between the grip member 8 and the grip-shaped portion 1a.

Operating the operation portion 3f in this manner makes it possible to arbitrarily move the main shaft member 5 in the longitudinal direction, thereby making the clamp apparatus grip each type of infusion stand. In addition, the meshed state is not released unless immediately after the rotating knob 9 is rotated, and the operation portion 3f cannot be operated. This prevents the syringe pump 22 from vertically moving, thus providing safety design. A mechanical means for implementing this will be described below.

As shown in Fig. 3, the cylindrical female thread member 2 made of stainless steel and having an inner diameter of about 10 mm and an outer diameter of about 16 mm is fixed to the upright shaped portion 1b of the clamp base 1 with screws 13 through the female thread portions. The main shaft member 5 is provided to be movable in the longitudinal direction relative to the cylindrical female thread member 2. The return coil spring 6 (see Fig. 2) is provided between the rotating knob 9 and the operation portion 3f.

More specifically, as shown in Fig. 4 which is an exploded perspective view of the arrangement of the main part, the tubular member 3 is fixed to the operation means 103. The tubular member 3 has an insertion port 3d for receiving the main shaft member 5 and the inner diameter portion 3c larger than the outer diameter of the main shaft member 5. The opening portions 3a on which the abutment surfaces 3b are formed are formed in the outer peripheral surface of the tubular member 3. The male thread pieces 4 each are made of a stainless resin, metal, or a material having similar strength, and is obtained by integrally forming the male thread portion 4a which is a double-start thread meshed with the female thread portion 2a, the second inclined surface 4b continuously formed from the corresponding male thread portion 4a, and the projection 4c for accommodating the built-in spring 7. The male thread pieces 4 are set, together with the built-in spring 7, in the housing portion 5w of the main shaft member 5, which has the first inclined surfaces 5a symmetrically formed in the vertical direction.

With the above assembly, the male thread pieces 4 are biased in the longitudinal direction by the effect of the built-in spring 7, and the inclined surfaces 4b slide on the corresponding inclined surfaces 5a. As a consequence, each of the male thread pieces 4 moves toward the corresponding female thread portion 2a. Assume that the built-in spring 7 has characteristics defined by an outer diameter of 3.55 to 10 mm, a length of 10 to 14 mm, and a spring modulus of 16 to 30 kgf/mm. This is because if the spring modulus is small, the pressing force against each of the male thread pieces 4 becomes small, whereas if the spring modulus is large, it is difficult to release each of the male thread pieces 4 from the corresponding female thread portion 2a.

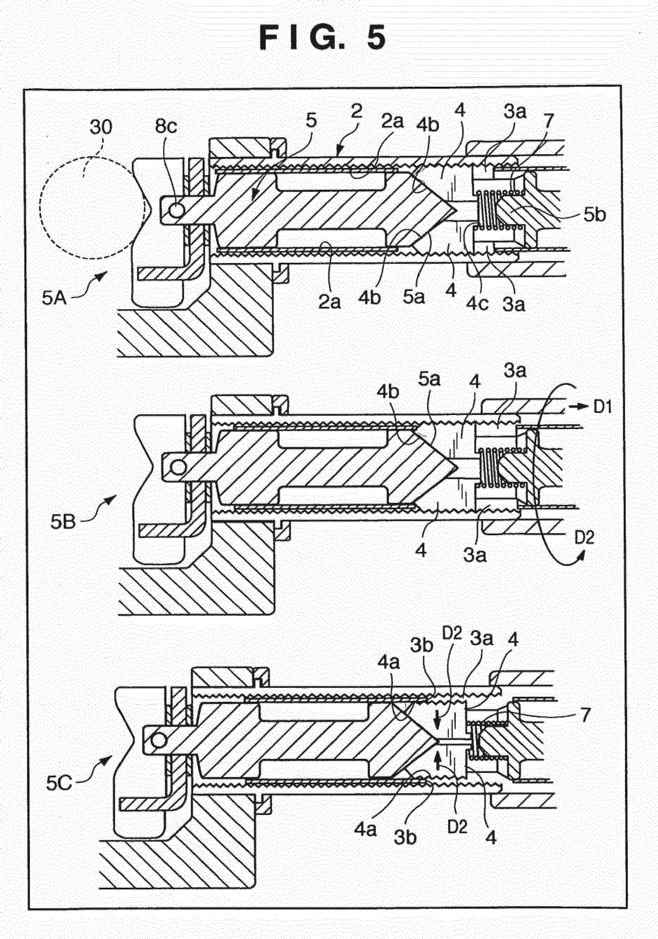

Fig. 5 shows sectional views taken along a line Y - Y in Fig. 4, each schematically showing how an operation means is fixed and released to and from the pole. 5A of Fig. 5 is a sectional view of the main part, showing a fixed (gripped) state for an explanation of operation. 5B of Fig. 5 is a sectional view of the main part, showing a midway state. 5C of Fig. 5 is a sectional view of the main part, showing a moving state for an explanation of operation.

As shown in 5A of Fig. 5, while the operation means is fixed to the pole (gripped state), the grip member 8 is firmly fixed to the pole 30, and the male thread portions 4a of the male thread pieces 4 set in the main shaft member 5 are meshed with the female thread portions 2a, respectively. As the rotating knob 9 is pivoted in the direction indicated by the arrow D2 in this state, the meshed state between the male thread portions 4a of the male thread pieces 4 and the female thread portions 2a is released. Subsequently, as shown in 5B of Fig. 5, when the user moves the operation portion 3f of the operation means 103 in the direction indicated by the arrow D1, the abutment surfaces 3b of the opening portions 3a of the tubular member 3 come into contact with the male thread portions 4a, respectively. This causes the male thread pieces 4 to move in the rightward direction along the inclined surfaces 5a of the main shaft member 5, thereby generating component force in the direction indicated by the arrow D2 in Fig. 5.

When the operation portion 3f further moves, the male thread pieces 4 are removed from the opening portions 3a. As described above, the tubular member 3 functions as a shutter for retracting the male thread pieces 4. As a consequence, the male thread portions 4a are fully retracted from the cylindrical outer diameter surface of the tubular member 3. When fixing the clamp apparatus to the pole 30 having a smaller outer diameter, the user moves the operation portion 3f of the operation means 103 as shown in Fig. 5 while the operation portion 3f is pulled to the rotating knob 9 side, thereby retracting the male thread pieces 4. When the user releases the operation means 103, the male thread pieces 4 protrude.

With the above arrangement, the user can freely move the rotating knob 9 back and forth by pulling the operation portion 3f of the operation means 103 to the rotating knob 9 side. This makes it possible to make adjustment in accordance with the diameter of the pole 30 to which the clamp apparatus is to be held. Slightly rotating the rotating knob 9 at a desired position can make the clamp apparatus reliably grip the pole 30.

When detaching the clamp apparatus from the pole 30, the user can easily operate the rotating knob 9 back and forth by only pulling the operation portion 3f of the operation means 103 to the rotating knob 9 side after slightly rotating the rotating knob 9 to loosen the screw. This makes it possible to eliminate the loss in operation time and shorten the time during which the operator holds a pump or the like. This also reduces the physical load.

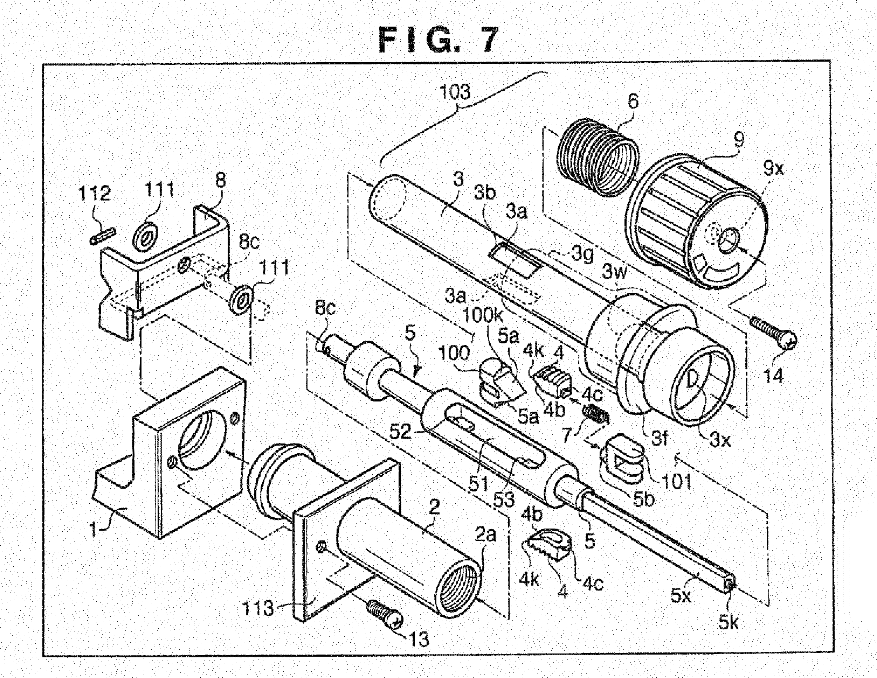

Fig. 6 is a sectional view of the main part, showing how a clamp apparatus 20 according to another embodiment of the present invention is directly fixed to the rear surface of a peristaltic infusion device 122. Fig. 7 is an exploded perspective view of the clamp apparatus 20 in Fig. 6. The same reference numerals as in Figs. 6 and 7 denote the same arrangements and parts as those which have already been described, and a repetitive description will be omitted.

As shown in Figs. 6 and 7, the diameter of a pole 30 falls within the range from d1 to d3. A tubular thread member 2 is vertically disposed at a grip-shaped portion on a surface on the side opposite to the side where an upright shaped portion is provided. Female thread portions 2a are formed on the inner peripheral surface throughout a length corresponding to the diameters d1 to d3. In addition, a main shaft member 5 has a pair of male thread pieces 4 built in a housing portion 51. The pair of male thread pieces 4 set a meshed state and an unmeshed state with respect to the female thread portion 2a. A grip member 8 has washers 111 set on its two surfaces. A slip pin 112 is then forced into a hole portion of a distal end 8c to pivotally support the grip member 8 on the main shaft member 5.

An operation portion 3f of an operation means 103 is disposed on the main shaft member 5 at the same axial position as that of a rotating knob 9 so as to allow the rotating knob 9 to move between an operation position which is the position of the rotating knob 9 when moving operation is performed for it and a separate position which is the position of the rotating knob 9 when no moving operation is performed for it. The operation portion 3f is also configured to set the pair of male thread pieces 4 in an unmeshed state when the rotating knob 9 is slightly pivoted to loosen the screw, and the operation portion 3f of the operation means 103 is pulled to the rotating knob 9 side.

A D-cut surface 5x shown in Fig. 7 is formed on the main shaft member 5. The D-cut surface 5x extends through a D-hole portion 3x formed by D-cutting the operation portion 3f and a D-hole portion 9x of the rotating knob 9. The operation means 103 can move to the rotating knob 9 side and is always biased in the direction to separate from the rotating knob 9 by a return spring 6. That is, the return spring 6 is provided to bias the operation means 103 toward the separate position spaced apart from the rotating knob 9. With the above arrangement, the pivoting force of the rotating knob 9 is transmitted to the main shaft member 5.

In addition, the housing portion 51 of the main shaft member 5 is formed as a through-space portion along the longitudinal direction of the main shaft member, as shown in Fig. 7. A first block member 100 having a pair of inclined surfaces 5a symmetrically formed to extend from the outer peripheral surface of the main shaft member 5 to the other end and a second block member 101 having a first projection 5b are separately arranged in the through-space portion, and are set in the through-space portion 51, together with the pair of male thread pieces 4 and the built-in spring 7. This facilitates assembly.

The pair of male thread pieces 4 are obtained by integrally forming male thread portions 4a respectively meshed with the female thread portions 2a, a pair of second inclined surfaces 4b which slide on a pair of first inclined surfaces 5a, respectively, and second projections 4c. The pair of male thread pieces 4 are set with respect to shaped portions 52 and 53 so as to prevent the escape of the male thread pieces 4 while a built-in spring 7 is provided between the first projection 5b and the second projections 4c to move and bias the pair of male thread pieces 4 toward the female thread portion 2a.

In addition, the tubular member 3 is configured to cover the outer peripheral surface of the main shaft member 5. Opening portions 3a are formed in the upper and lower surfaces of the tubular member 3. The pair of male thread pieces 4 respectively protrude in the opening portions 3a to be set in a meshed state.

Note that the pair of opening portions 3a formed in the tubular member 3 each have a rectangular shape as shown in Fig. 7. First abutment surfaces 3b are formed on the left side surfaces of the opening portions 3a, respectively. Second abutment surfaces 4k which come into contact with the first abutment surfaces, respectively, are formed from the left end sides of the female thread portions 4a of the pair of male thread pieces 4. With this arrangement, the male thread pieces 4 come into contact with abutment surfaces 100k of the first block member 100, thereby restricting the further movement of the male thread pieces 4. The first and second abutment surfaces are respectively formed within planes perpendicular to the axis of the main shaft member. This allows the stopper function (to be described later) to normally function. Note that it is possible to provide a ring-like member in place of the abutment surfaces 100k to implement a stopper function.

8A of Fig. 8 is a sectional view of the main part in Fig. 6, showing how the pair of male thread pieces 4 are meshed with the female thread portions 2a, respectively. 8B of Fig. 8 is a sectional view of the main part in Fig. 6, showing how the pair of male thread pieces 4 are unmeshed from the female thread portions 2a, respectively, by pivoting the rotating knob 9 and further moving the operation portion 3f of the operation means 103 in the direction indicated by an arrow D1. 8C of Fig. 8 is an enlarged sectional view immediately after the operation portion 3f of the operation means 103 is moved in the direction indicated by the arrow D1.

The state shown in 8A of Fig. 8 is set while the infusion device is used. In this state, the pair of male thread pieces 4 are meshed with the female thread portions 2a, respectively. For this reason, the clamp apparatus is gripping the pole. When the operation portion 3f of the operation means 103 is moved in the direction indicated by the arrow D1 in this state as shown in 8B of Fig. 8, the second abutment surfaces 4k formed from the left end sides of the female thread portions 4a come into contact with the first abutment surfaces 3b of the opening portions 3a, as shown in 8C of Fig. 8. This inhibits the operation means 103 from further moving in the rightward direction. Subsequently, when the rotating knob 9 is pivoted in the direction indicated by an arrow D3 which is a disengaging direction, an unmeshed state is set as shown in 8B of Fig. 8. Fig. 9 is a schematic view showing the relationship between the pole 30 and the grip member 8, the relationship between the male thread pieces 4 and the female thread portion 2a, the contents of manual operation, and the states of the clamp apparatus along with the sequence of steps S1 to S9. The same reference numerals as in this schematic view denote the same arrangements and parts as those which have already been described, and a repetitive description will be omitted.

In step S1 in which the infusion device is used, the grip member 8 is gripping the pole 30, and the male thread pieces 4 are meshed with the female thread portions 2a, respectively. In step S2, the rotating knob 9 is turned in the counter-clockwise direction (disengagement direction). Subsequently, in step S3, the operation portion 3f is moved to the side of the rotating knob 9 in the rightwards direction. As described with reference to 8C of Fig. 8, even if the operation portion 3f is moved to the right before the rotating knob is turned in the disengagement direction, the second abutment surfaces 4k formed from the left end sides of the male thread portions 4a then come into contact with the first abutment surfaces 3b of the opening portions 3a and stop. This inhibits the operation means 103 from further moving in the rightward direction. With the above arrangement, even if the user unintentionally pulls the operation portion 3f, the meshed state between the male thread pieces 4 and the female thread portions 2a is maintained to maintain the gripped state.

In step S3, the operating portion 3f is moved to the side of the rotating knob 9 in the rightwards direction, and the male thread pieces 4 are unmeshed from the female thread portions 2a. Thereafter, the state in which the pole 30 is gripped by the grip member 8 is released for the first time. The user then holds the infusion device with his/her hand. In step S4, the user moves the overall operation means 103 to the right to detach the clamp apparatus from the pole. In step S5, the user sets the clamp apparatus to another pole. In step S6, the user prepares for a meshed state by returning the operation portion 3f after moving the overall operation means 103 to the right.

In step S7, the user pivots the rotating knob 9 clockwise to set a meshed state so as to set a gripped state. In step S8, the user fixes the rotating knob 9. In step S9, the user prepares for the use of an infusion.

As described above, according to this embodiment, when fixing an infusion device to the pole of the infusion stand or detaching the device from the pole of the infusion stand, the user need not repeatedly and continuously rotate the rotating knob while holding the infusion device from the beginning. This makes it possible to minimize the time loss and physical load. In addition, in this embodiment, the meshed state is maintained immediately after the operation portion is moved to the operation position, and is released for the first time when the rotating knob is pivoted. This allows the user to safely and reliably detach an infusion device from the pole of the stand. As described above, this embodiment provides a clamp apparatus which is beneficial especially for physically weak attendants.

The present invention is not limited to the above embodiments and various changes and modifications can be made within the scope of the present invention. Therefore, to apprise the public of the scope of the present invention, the following claims are made.

## Claims

1. A clamp apparatus (20) for poles of infusion stands with different diameters, comprising a clamp base (1) comprising a grip-shaped portion (1a) which grips one outer peripheral surface of each of the poles and an upright shaped portion (1b) provided at a position to oppose the grip-shaped portion (1a), and a main shaft member (5) having one end rotatably provided with a grip member (8) which grips the other outer peripheral surface of the pole, and the other end to which a rotating knob (9) for rotating operation is fixed, and sets a gripped state and an ungripped state relative to the pole by performing rotating operation for the rotating knob (9) clockwise or counterclockwise and moving the grip member (8), **characterized in that** the clamp apparatus further comprises:
a tubular thread member (2) which is vertically disposed at the upright shaped portion (1b) on a surface on a side opposite to a side where the grip-shaped portion (1a) is provided, has an inner peripheral surface on which female thread portions (2a) are formed throughout a length corresponding to the diameters of the respective poles, and through which the main shaft member (5) extends;
a pair of male thread pieces (4, 4) configured to be set in a meshed state or an unmeshed state relative to the female thread portions (2a) and built in a housing portion of the main shaft member (5); and
an operation means (103) which comprises an operation portion (3f) which is mounted on the main shaft member (5) at an axial position of the rotating knob (9) so as to be movable between an operation position where the rotating knob (9) can be moved back and forth and a separate position where the rotating knob (9) cannot be moved back and forth, and which sets said pair of male thread pieces (4, 4) in the unmeshed state when the operation portion (3f) is moved to the operation position.

2. The clamp apparatus according to claim 1, wherein
the housing portion (51) of the main shaft member (5) is formed as a through-space portion along a longitudinal direction of the main shaft member,
a pair of first inclined surfaces (5a) are formed on a left side surface side of the through-space portion so as to be symmetric with respect to a direction extending from the outer peripheral surface of the main shaft member to the other end of the main shaft member, and a first projection (5b) is formed on a right side surface side of the through-space portion,
male thread portions (4, 4) respectively meshed with the female thread portions (2a), a pair of second inclined surfaces (4b, 4b) which slide on the pair of first inclined surfaces, and second projections (4c) are integrally formed on said pair of male thread pieces,
a built-in spring (7) is provided between the first projection and the second projections to bias said pair of male thread pieces (4,4) in a direction toward the female thread portions (2a), and
said operation means comprises a tubular member (3) which covers the outer peripheral surface of the main shaft member and in which a pair of opening portions (3a) for setting said pair of male thread pieces in the meshed state are formed, and a return spring (6) which biases the operation portion (3f) in a direction toward the separate position.

3. The clamp apparatus according to claim 2, wherein first abutment surfaces are formed on left side surfaces of the pair of opening portions (3a) of the tubular member,
second abutment surfaces which come into contact with the first abutment surfaces from left end sides of the female thread portions (2a) to the first abutment surfaces are formed in said pair of male thread pieces (4,4), and
the first abutment surfaces come into contact with the second abutment surfaces, immediately after the operation portion (3a) moves to the operation position, to maintain the meshed state, and the unmeshed state is set when the rotating knob (9) is rotated afterward.

4. The clamp apparatus according to claim 3, wherein the first abutment surfaces and the second abutment surfaces are respectively formed within planes perpendicular to an axis of the main shaft member (5).

5. The clamp apparatus according to claim 2, wherein a first block member (100) on which the pair of first inclined surfaces (5a) are formed and a second block member (101) on which the first projection (5b) is formed are discrete, and the first block member and the second block member are set in the through-space portion, together with said pair of male thread pieces (4,4) and the built-in spring (7).

6. The clamp apparatus according to claim 1, wherein a mount base (21) for an infusion device is used while being fixed on the clamp base (1).

7. The clamp apparatus according to claim 1, wherein said operation means comprises a boot cover (3g) made of an elastic material which prevents entrance of a drug by covering said tubular screw member.

8. The clamp apparatus according to claim 1, wherein the clamp base (1) is used while being directly fixed to a rear surface of an infusion device.

## Patentansprüche

1. Klemmvorrichtung (20) für Stangen von Infusionsständern mit unterschiedlichen Durchmessern, die ein Klemmstück (1), das einen griffförmigen Abschnitt (1a), der eine Außenumfangsfläche jeder der Stangen greift, und einen senkrecht geformten Abschnitt (1b) umfasst, der an einer dem griffförmigen Abschnitt (1a) gegenüberliegenden Position bereitgestellt ist und ein Hauptschaftelement (5) umfasst, das ein Ende, das mit einem Griffelement (8) drehbar vorgesehen ist, das die andere Außenumfangsfläche der Stange greift und das andere Ende aufweist, an dem ein Drehknopf (9) für Drehbetätigung befestigt ist, und das einen Greif- und einen gelösten Greif-Zustand in Bezug auf die Stange einstellt, indem eine Drehbetätigung im Uhrzeigersinn und gegen den Uhrzeigersinn für den Drehknopf (9) durchgeführt und indem das Griffelement (g) bewegt wird, **dadurch gekennzeichnet, dass** die Klemmvorrichtung weiters Folgendes umfasst:
ein Rohrgewindeelement (2), das vertikal an dem senkrecht geformten Abschnitt (1b) auf einer Oberfläche auf einer Seite angeordnet ist, die einer Seite, an der der griffförmige Abschnitt (1a) bereitgestellt ist, gegenüberliegt, das eine Innenumfangsfläche aufweist, auf der Innengewindeabschnitte (2a) über eine Länge geformt sind, die den Durchmessern der entsprechenden Stangen entspricht und durch die sich das Hauptschaftelement (5) erstreckt;
ein Außengewindestückpaar (4, 4), das konfiguriert ist, um in einen Kämmeingriff-Zustand oder einen Außer-Kämmeingriff-Zustand in Bezug auf die Innengewindeelemente (2a) eingestellt zu werden und die in einem Gehäuseteil des Hauptschaftelements (5) eingebaut sind; und
eine Betätigungseinrichtung (103), die einen Betätigungsabschnitt (3f) umfasst, der an einer Axialposition des Drehknopfs (9) auf dem Hauptschaftelement (5) montiert ist, sodass er zwischen einer Betätigungsposition, an der der Drehknopf (9) vor und zurück bewegt werden kann und einer separaten Position, an der der Drehknopf (9) nicht vor und zurück bewegt werden kann, bewegbar ist und die das Außengewindestückpaar (4, 4) in den Außer-Kämmeingriff-Zustand versetzt, wenn der Betätigungsabschnitt (3f) an die Betätigungsposition bewegt wird.

2. Klemmvorrichtung nach Anspruch 1, wobei
der Gehäuseteil (51) des Hauptschaftelements (5) als Durchgangsraumabschnitt entlang einer Längsrichtung des Hauptschaftelements ausgebildet ist,
ein Paar von ersten geneigten Oberflächen (5a) an einer linken Seitenoberflächen-Seite des Durchgangsraumabschnitts ausgebildet sind, sodass sie symmetrisch in Bezug auf eine Richtung sind, die sich von der AußenumfangsOberfläche des Hauptschaftelements zum anderen Ende des Hauptschaftelements erstreckt und eine erste Nase (5b) an einer rechten Seitenoberflächen-Seite des Durchgangsraumabschnitts ausgebildet ist,
Außengewindeabschnitte (4, 4), die jeweils mit den Innengewindeabschnitten (2a) in Kämmeingriff sind, ein Paar von zweiten geneigten Oberflächen (4b, 4b), das sich auf das Paar von ersten geneigten Oberflächen schiebt und zweite Nasen (4c) einstückig auf den Außengewindestücken ausgebildet sind,
eine eingebaute Feder (7) zwischen der ersten Nase und den zweiten Nasen bereitgestellt ist, um das Außengewindestückpaar (4, 4) in eine Richtung hin zu den Innengewindeabschnitten (2a) vorzuspannen und
die Betätigungseinrichtung ein Rohrelement (3), das die AußenumfangsOberfläche des Hauptschaftelements bedeckt und in dem ein Paar von Öffnungsabschnitten (3a) zum Versetzen des Außengewindestückpaars in den Kämmeingriff-Zustand ausgebildet sind, und eine Rückstellfeder (6) umfasst, die den Betätigungsabschnitt (3f) in eine Richtung hin zu der separaten Position vorspannt.

3. Klemmvorrichtung nach Anspruch 2, wobei erste Anlageflächen auf linken Seitenflächen des Paars von Öffnungsabschnitten (3a) des Rohrelements ausgebildet sind,
zweite Anlageflächen, die mit den ersten Anlageflächen von linken Endseiten der Innengewindeabschnitte (2a) zu den ersten Anlageflächen in Berührung kommen, in dem Außengewindestückpaar (4, 4) gebildet sind und
die ersten Anlageflächen mit den zweiten Anlageflächen unmittelbar nachdem sich der Betätigungsabschnitt (3a) zu der Betätigungsposition bewegt, in Berührung kommen, um den Kämmeingriff-Zustand aufrechtzuerhalten und der Außer-Kämmeingriff-Zustand eingestellt wird, wenn darauffolgend der Drehknopf (9) gedreht wird.

4. Klemmvorrichtung nach Anspruch 3, wobei die ersten Anlageflächen und die zweiten Anlageflächen jeweils innerhalb von Ebenen gebildet sind, die normal auf eine Achse des Hauptschaftselements (5) stehen.

5. Klemmvorrichtung nach Anspruch 2, wobei ein erstes Blockelement (100), auf dem das Paar von ersten geneigten Oberflächen (5a) ausgebildet ist und ein zweites Blockelement (101), auf dem die erste Nase (5b) ausgebildet ist, separat sind, und das erste Blockelement und das zweite Blockelement gemeinsam mit dem Außengewindestückpaar (4, 4) und der eingebauten Feder (7) in dem Durchgangsraumabschnitt eingestellt sind.

6. Klemmvorrichtung nach Anspruch 1, wobei ein Montagestück (21) für eine Infusionsvorrichtung verwendet wird, während es auf dem Klemmstück (1) befestigt ist.

7. Klemmvorrichtung nach Anspruch 1, wobei die Betätigungseinrichtung eine Manschettenabdeckung (3g) aus einem elastischen Material umfasst, die das Eindringen eines Arzneimittels durch Abdecken des röhrenförmigen Schraubenelements verhindert.

8. Klemmvorrichtung nach Anspruch 1, wobei das Klemmstück (1) verwendet wird, während es direkt an der hinteren Oberfläche einer Infusionsvorrichtung befestigt ist.

## Revendications

1. Dispositif de serrage (20) pour des poteaux de supports de perfusion de différents diamètres, comprenant une base de serrage (1) comprenant une partie en forme de pince (1a) qui saisit une surface périphérique externe de chacun des poteaux et une partie de forme droite (1b) prévue dans une position pour s'opposer à la partie en forme de pince (1a), et un élément d'arbre principal (5) ayant une première extrémité munie de manière rotative d'un élément de préhension (8) qui saisit l'autre surface périphérique extérieure du poteau, et à l'autre extrémité duquel est fixé un bouton rotatif (9) pour une opération de rotation, et qui établit un état de préhension et un état de non-préhension par rapport au poteau en effectuant une opération de rotation pour le bouton rotatif (9) dans le sens des aiguilles d'une montre et dans le sens inverse des aiguilles d'une montre et en déplaçant l'élément de préhension (8), **caractérisé en ce que** le dispositif de serrage comprend en outre :
un élément de filet tubulaire (2) qui est disposé verticalement au niveau de la partie de forme droite (1b) sur une surface sur un côté opposé à un côté où la partie en forme de pince (1a) est prévue, présente une surface périphérique interne sur laquelle des parties de filet femelles (2a) sont formées sur une longueur correspondant aux diamètres des poteaux respectifs, et à travers laquelle s'étend l'élément d'arbre principal (5) ;
une paire de parties de filet mâles (4, 4) configurées pour être réglées dans un état d'engrènement ou un état dégagé par rapport aux parties de filet femelles (2a) et intégrées dans une partie de logement de l'élément d'arbre principal (5) ; et
un moyen d'actionnement (103) qui comprend une partie d'actionnement (3f) qui est montée sur l'élément d'arbre principal (5) dans une position axiale dans laquelle le bouton rotatif (9) de manière à pouvoir se déplacer entre une position d'actionnement dans laquelle le bouton rotatif (9) peut être déplacé en va-et-vient et une position de séparation dans laquelle le bouton rotatif (9) ne peut pas être déplacé en va-et-vient, et qui met ladite paire de parties de filet mâles (4, 4) dans l'état dégagé lorsque la partie d'actionnement (3f) est déplacée vers la position d'actionnement.

2. Dispositif de serrage selon la revendication 1, dans lequel
la partie de logement (51) de l'élément d'arbre principal (5) est formée comme une partie d'espace traversant le long d'une direction longitudinale de l'élément d'arbre principal,
une paire de premières surfaces inclinées (5a) sont formées sur un côté de surface latérale gauche de la partie d'espace traversant de manière à être symétriques par rapport à une direction s'étendant à partir de la surface périphérique externe de l'élément d'arbre principal jusqu'à l'autre extrémité de l'élément d'arbre principal, et une première saillie (5b) est formée sur un côté de surface latérale droite de la partie d'espace traversant,
des parties de filet mâles (4, 4) engrenant respectivement avec les parties de filet femelles (2a), une paire de secondes surfaces inclinées (4b, 4b) qui coulissent sur la paire de premières surfaces inclinées, et des secondes saillies (4c) sont formées intégralement sur ladite paire de parties de filet mâles,
un ressort intégré (7) est prévu entre la première saillie et les secondes saillies pour solliciter ladite paire de parties de filet mâles (4, 4) dans une direction vers les parties de filet femelles (2a), et
ledit moyen d'actionnement comprend un élément tubulaire (3) qui recouvre la surface périphérique externe de l'élément principal d'arbre et dans lequel sont formés une paire de parties d'ouverture (3a) pour régler ladite paire de parties de filet mâles dans l'état engrené, et un ressort de rappel (6) qui sollicite la partie d'actionnement (3f) dans une direction vers la position de séparation.

3. Dispositif de serrage selon la revendication 2, dans lequel des premières surfaces de butée sont formées sur les surfaces latérales gauches de la paire de parties d'ouverture (3a) de l'élément tubulaire,
des secondes surfaces de butée venant en contact avec les premières surfaces de butée à partir des côtés d'extrémité gauche des parties de filet femelles (2a) jusqu'aux premières surfaces de butée sont formées dans ladite paire de parties de filet mâles (4, 4), et
les premières surfaces de butée viennent en contact avec les secondes surfaces de butée, immédiatement après le déplacement de la partie d'actionnement (3a) vers la position d'actionnement, afin de maintenir l'état engrené, et l'état dégagé est activé lorsque le bouton rotatif (9) est tourné par la suite.

4. Dispositif de serrage selon la revendication 3, dans lequel les premières surfaces de butée et les secondes surfaces de butée sont respectivement formées dans des plans perpendiculaires à un axe de l'élément d'arbre principal (5).

5. Dispositif de serrage selon la revendication 2, dans lequel un premier bloc (100) sur lequel est formée la paire de premières surfaces inclinées (5a) et un second bloc (101) sur lequel est formée la première saillie (5b) sont distincts, et le premier élément de bloc et le second élément de bloc sont placés dans la partie d'espace traversant, conjointement avec ladite paire de pièces de filet mâles (4, 4) et le ressort intégré (7).

6. Dispositif de serrage selon la revendication 1, dans lequel une base de montage (21) pour un dispositif de perfusion est utilisée tout en étant fixée sur la base de serrage (1).

7. Dispositif de serrage selon la revendication 1, dans lequel ledit moyen d'actionnement comprend un recouvrement de protection (3g) constitué d'un matériau élastique qui empêche l'entrée d'un médicament en recouvrant ledit élément de vis tubulaire.

8. Dispositif de serrage selon la revendication 1, dans laquelle la base de serrage (1) est utilisée tout en étant directement fixée à la surface arrière d'un dispositif de perfusion.
